# EUROPÄISCHE PATENTSCHRIFT

(11) **EP 0 942 706 B1**
(45) Veröffentlichungstag und Bekanntmachung des Hinweises auf die Patenterteilung: **23.03.2005**
(21) Anmeldenummer: 97950090.7
(22) Anmeldetag: 04.11.1997
(51) Int. Cl.: A61K 7/06, A61K 7/50, C11D 1/94

(54) **WÄSSRIGE PERLGLANZKONZENTRATE**
AQUEOUS PEARLY LUSTRE CONCENTRATES
CONCENTRES AQUEUX DE LUSTRE PERLAIRE

(30) Priorität: 13.11.1996 DE 19646882
(43) Veröffentlichungstag der Anmeldung: 22.09.1999
(73) Patentinhaber: Cognis Deutschland GmbH & Co. KG, 40589 Düsseldorf (DE)
(72) Erfinder: WILHELM, Josef, D-36088 Hünfeld (DE); BIMCZOK, Rudolf, D-64342 Seeheim-Jugenheim (DE); KOHL, Werner, D-36088 Hünfeld (DE); ANSMANN, Achim, D-40699 Erkrath (DE); KAWA, Rolf, D-40789 Monheim (DE)
(86) Internationale Anmeldenummer: PCT/EP1997/006084
(87) Internationale Veröffentlichungsnummer: WO 1998/020844

(56) Entgegenhaltungen:
- EP-A- 0 568 848
- WO-A-93/18737
- WO-A-96/21711
- WO-A-96/30475
- WO-A-96/31586
- DE-A- 19 511 570

## Beschreibung

### Gebiet der Erfindung

Die Erfindung betrifft wäßrige, polyolfreie Perlglanzkonzentrate auf Basis ausgewählter tensidischer Emulgatoren und Perlglanzwachsen sowie deren Verwendung zur Herstellung perlglänzender oberflächenaktiver Zubereitungen.

### Stand der Technik

Der weich schimmernde Glanz von Perlen hat auf den Menschen schon seit Jahrtausenden eine besondere Faszination ausgeübt. Es ist daher kein Wunder, daß die Hersteller von kosmetischen Zubereitungen versuchen, ihren Produkten ein attraktives, wertvolles und gehaltvolles Erscheinungsbild zu verleihen. Der erste seit dem Mittelalter in der Kosmetik eingesetzte Perlglanz war eine perlglänzende Paste aus natürlichen Fischschuppen. Zu Anfang dieses Jahrhunderts entdeckte man, daß Wismutoxidchloride ebenfalls in der Lage sind, Perlglanz zu erzeugen. Für die moderne Kosmetik sind hingegen Perlglanzwachse, insbesondere vom Typ der Glycolmono- und -difettsäureester von Bedeutung, die überwiegend zur Erzeugung von Perlglanz in Haarshampoos und Duschgelen eingesetzt werden. Eine Übersicht zu modernen, perlglänzenden Formulierungen findet sich von A.Ansmann und R.Kawa in **Parf.Kosm. 75, 578 (1994).**

Aus dem Stand der Technik sind eine ganze Reihe von Perlglanzmitteln und -formulierungen bekannt. So werden beispielsweise in der **DE-A1 35 19 080** (Henkel) fließfähige Perlglanzkonzentrate beschrieben, die 5 bis 15 Gew.-% Glycolester, 1 bis 6 Gew.-% Fettsäuremonoethanolamide und 1 bis 5 Gew.-% nichtionische Ethylenoxidaddukte mit HLB-Werten im Bereich von 12 bis 18 aufweisen. Gegenstand der **DE-A1 37 24 547** (Henkel) sind alkanolamidfreie Perlglanzkonzentrate, die neben Glycolfettsäureestern 5 bis 20 Gew.-% Fettsäuren und 3 bis 10 Gew.-% Emulgatoren enthalten. In den Europäischen Patentschriften **EP-B1 0 376 083** und **EP-B1 0 570 398** (Henkel) wird vorgeschlagen, 15 bis 40 Gew.-% Glycolfettsäureester zusammen mit 5 bis 55 Gew.-% nichtionischer, ampholytischer oder zwitterionischer Emulgatoren und 0,1 bis 5 Gew.-% bzw. 15 bis 40 Glycerin zu einem Perlglanzkonzentrat zu verarbeiten. Aus der Schrift **DE-A1 42 24 715** (Hoechst) sind fließfähige, konservierungsmittelfreie Perlglanzdispersionen bekannt, die neben Glycolfettsäureestern, Tenside (Betaine, Aniontenside, Ethoxylate) und Glycerin enthalten. In der Europäischen Offenlegungsschrift **EP-A1 0 684 302** (Th.Goldschmidt) werden Perlglanzmittel mit einem Gehalt an Polyglycerinestern vorgeschlagen. In den beiden Europäischen Patenten **EP-B1 0 181 773** und **EP-B1 0 285 389** (Procter & Gamble) werden siliconhaltige Shampoozubereitungen offenbart, die als Perlglanzwachse langkettige Acylverbindungen enthalten. Die Verwendung von Alkylpolyglucosiden und ausgewählten weiteren Tensiden (Alkylsulfate, Fettsäureisethionate, Betaine und dergleichen) als Emulgatoren zur Herstellung von Perlglanzmitteln sind aus den Druckschriften **WO 93/15171** und **WO 95/03782** (ICI) sowie **WO 94/24248** (Henkel Corp.) und **WO 95/13863** (SEPPIC) bekannt. Schließlich werden in der Europäischen Offenlegungsschriften **EP-A1 0 367 939** (Wella) haarkonditionierende Zubereitungen mit Perlglanz mit ausgewählten kationischen Tensiden beschrieben.

Die WO-A-9 621 711 offenbart Perlglanzkonzentrate, welche beispielsweise Ethylenglycolmonostearat, Natrium Laurylethersulfat und Cocoamidopropylbetain enthalten.

Trotz dieses umfangreichen Stands der Technik besteht nach wie vor das Problem, wäßrige Perlglanzkonzentrate beispielsweise als Rohstoffe für die Herstellung von Haarshampoos zur Verfügung zu stellen, die auch ohne Mitverwendung von Polyolen (beispielsweise Glycerin) hochkonzentriert fließfähig sind. Die Aufgabe der Erfindung hat somit darin bestanden, diesem Mangel abzuhelfen.

### Beschreibung der Erfindung

Gegenstand der Erfindung sind wäßrige Perlglanzkonzentrate, frei von Polyolen, enthaltend
(a) Alkylethersulfate,
(b1) Betaine und/oder
(b2) Alkyl- und/oder Alkenyloligoglykoside und
(c) (Oligo)Ethylenglycolmono- und/oder -difettsäureester.

Überraschenderweise wurde gefunden, daß eine Emulgatormischung aus ausgewählten anionischen und nichtionischen bzw. amphoteren Tensiden zusammen mit Perlglanzwachsen die Herstellung von hochkonzentriert fließfähigen, wäßrigen Perlglanzkonzentraten erlaubt, ohne daß hierzu die Mitverwendung von Polyolen wie beispielsweise Glycerin erforderlich ist. Die Erfindung schließt dabei die Erkenntnis ein, daß die Komponenten (a) und (b) flüssigkristalline Phasen bilden.

### Alkylethersulfate

Alkylethersulfate stellen bekannte anionische Tenside dar, die großtechnisch durch SO₃- oder Chlorsulfonsäure (CSA)-Sulfatierung von Fettalkoholpolyglycolethern und nachfolgende Neutralisation hergestellt werden. Im Sinne der Erfindung kommen Ethersulfate in Betracht, die der Formel **(I)** folgen,

**R**^{**1**}**O-(CH**_{**2**}**CH**_{**2**}**O)**_{**m**}**SO**_{**3**}**X (I)**

in der R¹ für einen linearen oder verzweigten Alkyl- und/oder Alkenylrest mit 6 bis 22 Kohlenstoffatomen, m für Zahlen von 1 bis 10 und X für ein Alkali- und/oder Erdalkalimetall, Ammonium, Alkylammonium, Alkanolammonium oder Glucammonium steht. Typische Beispiele sind die Sulfate von Anlagerungsprodukten von durchschnittlich 1 bis 10 und insbesondere 2 bis 5 Mol Ethylenoxid an Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol und Brassidylalkohol sowie deren technische Mischungen, in Form ihrer Natrium- und/oder Magnesiumsalze. Die Ethersulfate können dabei sowohl eine konventionelle als auch eine eingeengte Homologenverteilung aufweisen. Besonders bevorzugt ist der Einsatz von Ethersulfaten auf Basis von Addukten von durchschnittlich 2 bis 3 Mol Ethylenoxid an technische C_{12/14}- bzw. C_{12/18}- Kokosfettalkoholfraktionen in Form ihrer Natrium- und/oder Magnesiumsalze.

### Betaine

Betaine stellen bekannte Tenside dar, die überwiegend durch Carboxyalkylierung, insbesondere Carboxymethylierung von aminischen Verbindungen hergestellt werden. Vorzugsweise werden die Ausgangsstoffe mit Halogencarbonsäuren oder deren Salzen, insbesondere mit Natriumchloracetat kondensiert, wobei pro Mol Betain ein Mol Salz gebildet wird. Ferner ist auch die Anlagerung von ungesättigten Carbonsäuren wie beispielsweise Acrylsäure möglich. Zur Nomenklatur und insbesondere zur Unterscheidung zwischen Betainen und "echten" Amphotensiden sei auf den Beitrag von U.Ploog in **Seifen-Öle-Fette-Wachse, 198, 373 (1982)** verwiesen. Weitere Übersichten zu diesem Thema finden sich beispielsweise von A.O'Lennick et al. in **HAPPI, Nov. 70 (1986),** S.Holzman et al. in **Tens. Surf.Det. 23, 309 (1986),** R.Bibo et al. in **Soap Cosm.Chem.Spec.Apr. 46 (1990)** und P.Ellis et al. in **Euro Cosm. 1, 14 (1994).** Beispiele für geeignete Betaine stellen die Carboxyalkylierungsprodukte von sekundären und insbesondere tertiären Aminen dar, die der Formel **(II)** folgen, 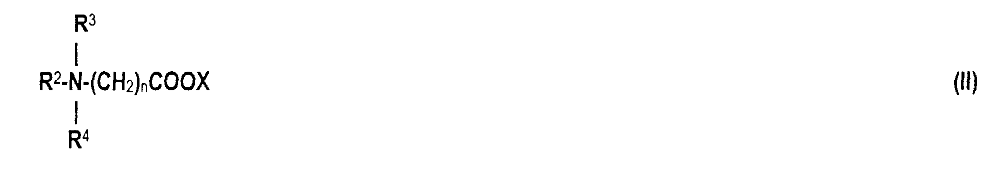 in der R² für Alkyl- und/oder Alkenylreste mit 6 bis 22 Kohlenstoffatomen, R³ für Wasserstoff oder Alkylreste mit 1 bis 4 Kohlenstoffatomen, R⁴ für Alkylreste mit 1 bis 4 Kohlenstoffatomen, n für Zahlen von 1 bis 6 und X für ein Alkali- und/oder Erdalkalimetall oder Ammonium steht. Typische Beispiele sind die Carboxymethylierungsprodukte von Hexylmethylamin, Hexyldimethylamin, Octyldimethylamin, Decyldimethylamin, Dodecylmethylamin, Dodecyldimethylamin, Dodecylethylmethylamin, C_{12/14}-Kokosalkyldimethylamin, Myristyldimethylamin, Cetyldimethylamin, Stearyldimethylamin, Stearylethylmethylamin, Oleyldimethylamin, C_{16/18}-Talgalkyldimethylamin sowie deren technische Gemische.

Weiterhin kommen auch Carboxyalkylierungsprodukte von **Amidoaminen** in Betracht, die der Formel **(III)** folgen, 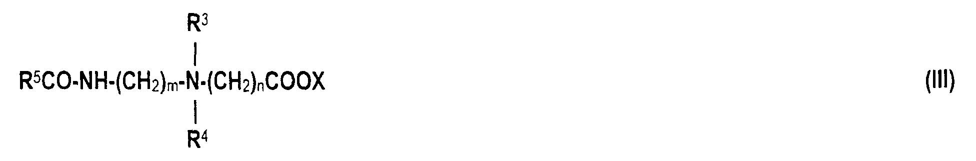 in der R⁵CO für einen aliphatischen Acylrest mit 6 bis 22 Kohlenstoffatomen und 0 oder 1 bis 3 Doppelbindungen, m für Zahlen von 1 bis 3 steht und R³, R⁴, n und X die oben angegebenen Bedeutungen haben. Typische Beispiele sind Umsetzungsprodukte von Fettsäuren mit 6 bis 22 Kohlenstoffatomen, namentlich Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Gemische, mit N,N-Dimethylaminoethylamin, N,N-Dimethylaminopropylamin, N,N-Diethylaminoethylamin und N,N-Diethylaminopropylamin, die mit Natriumchloracetat kondensiert werden. Bevorzugt ist der Einsatz eines Kondensationsproduktes von C_{8/18}-Kokosfettsäure-N,N-dimethylaminopropylamid mit Natriumchloracetat.

Weiterhin kommen als geeignete Ausgangsstoffe für die im Sinne der Erfindung einzusetzenden Betaine auch Imidazoliniumbetaine in Frage, bei denen es sich ebenfalls um bekannte Stoffe handelt, die man beispielsweise durch cyclisierende Kondensation von 1 oder 2 Mol Fettsäure mit mehrwertigen Aminen wie beispielsweise Aminoethylethanolamin (AEEA) oder Diethylentriamin erhalten werden können. Die entsprechenden Carboxyalkylierungsprodukte stellen Gemische unterschiedlicher offenkettiger Betaine dar. Typische Beispiele sind Kondensationsprodukte der oben genannten Fettsäuren mit AEEA, vorzugsweise Imidazoline auf Basis von Laurinsäure oder wiederum C_{12/14}-Kokosfettsäure, die anschließend mit Natriumchloracetat betainisiert werden.

### Alkyl- und/oder Alkenyloligoglykoside

Alkyl- und Alkenyloligoglykoside stellen bekannte nichtionische Tenside dar, die der Formel **(IV)** folgen,

**R**^{**6**}**O-[G]**_{**p**} **(IV)**

in der R⁶ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden. Stellvertretend für das umfangreiche Schrifttum sei hier auf die Schriften **EP-A1 0 301 298** und **WO 90/03977** verwiesen.

Die Alkyl- und/oder Alkenyloligoglykoside können sich von Aldosen bzw. Ketosen mit 5 oder 6 Kohlenstoffatomen, vorzugsweise der Glucose ableiten. Die bevorzugten Alkyl- und/oder Alkenyloligoglykoside sind somit Alkyl- und/oder Alkenyloligo**glucoside**. Die Indexzahl p in der allgemeinen Formel **(IV)** gibt den Oligomerisierungsgrad (DP), d. h. die Verteilung von Mono- und Oligoglykosiden an und steht für eine Zahl zwischen 1 und 10. Während p in einer gegebenen Verbindung stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt.

Der Alkyl- bzw. Alkenylrest R⁶ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese erhalten werden. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3). Der Alkyl- bzw. Alkenylrest R⁶ kann sich ferner auch von primären Alkoholen mit 12 bis 22, vorzugsweise 12 bis 14 Kohlenstoffatomen ableiten. Typische Beispiele sind Laurylalkohol, Myristylalkohol, Cetylalkohol, Palmoleylalkohol, Stearylalkohol, lsostearylalkohol, Oleylalkohol, Elaidylalkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden können. Bevorzugt sind Alkyloligoglucoside auf Basis von gehärtetem C_{12/14}-Kokosalkohol mit einem DP von 1 bis 3.

### (Oligo)Ethylenglycolfettsäureester

Ethylenglycol- bzw. Oligoethylenglycolfettsäureester, die als Perlglanzwachse in Betracht kommen, folgen der Formel **(V),**

**R**^{**7**}**CO(OCH**_{**2**}**CH**_{**2**}**)**_{**z**}**OX (V)**

in der R⁷CO für einen linearen Acylrest mit 6 bis 22 Kohlenstoffatomen, X für Wasserstoff oder einen Acylrest R⁷CO und z für Zahlen von 1 bis 10, vorzugsweise 1 bis 3 steht. Typische Beispiele hierfür sind Ethylenglycolmonolaurat, Ethylenglycoldilaurat, Ethylenglycolmonopalmitat, Ethylenglycoldipalmitat, Ethylenglycolmonostearat, Ethylenglycoldistearat, Diethylenglycolmonostearat, Diethylenglycoldistearat und deren technischen Mischungen sowie vorzugsweise Triethylenglycolmono- und/oder -distearat. Falls gewünscht, können die Ester mit weiteren bekannten Perlglanzwachsen, vorzugsweise Fettsäurealkanolamiden, abgemischt werden, wobei das Gewichtsverhältnis unkritisch ist und im Bereich 90 : 10 bis 10 : 50 und vorzugsweise 40 : 60 bis 60 : 40 liegen kann.

### Perlglanzkonzentrate

Perlglanzkonzentrate, die im Sinne der Erfindung besonders bevorzugt sind, weisen folgende allgemeine Zusammensetzung auf:
(a) 20 bis 80, vorzugsweise 30 bis 70 Gew.-% Alkylethersulfate,
(b1) 1 bis 30, vorzugsweise 5 bis 20 Gew.-% Betaine und/oder
(b2) 5 bis 30, vorzugsweise 5 bis 20 Gew.-% Alkyl- und/oder Alkenyloligoglykoside und
(c) 1 bis 10, vorzugsweise 2 bis 8 Gew.-% (Oligo)Ethylenglycolmono- und/oder -difettsäureester,
mit der Maßgabe, daß sich die Mengenangaben gegebenenfalls mit Wasser und Elektrolytsalzen zu 100 Gew.-% ergänzen. Vorzugsweise werden die Komponenten (a) und (b) im Gewichtsverhältnis 5 : 1 bis 20 : 1, die Komponenten (a) und (c) im Gewichtsverhältnis 5 : 1 bis 30 : 1 und die Komponenten (b) und (c) im Gewichtsverhältnis 1 : 1 bis 10 : 1 eingesetzt. Insbesondere kann das Gewichtsverhältnis der Komponenten (a) : (b) : (c) im Bereich (15 bis 25) : (2 bis 4) : (0,5 bis 2) liegen. Die Perlglanzkonzentrate enthalten üblicherweise 10 bis 70 vorzugsweise 30 bis 60 und insbesondere 40 bis 50 Gew.-% Wasser sowie gegebenenfalls einen Anteil von 1 bis 6 und vorzugsweise 2 bis 5 Gew.-% Elektrolytsalze wie beispielsweise Natriumchlorid oder Magnesiumchlorid zur Einstellung der Viskosität.

### Gewerbliche Anwendbarkeit

Ein weiterer Gegenstand der Erfindung betrifft die Verwendung der wäßrigen Perlglanzkonzentrate zur Herstellung von perlglänzenden oberflächenaktiven Zubereitungen wie beispielsweise Haarshampoos oder manuellen Geschirrspülmitteln.

Die fertigen Zubereitungen können die erfindungsgemäßen Perlglanzkonzentrate in Mengen von 0,1 bis 30 und vorzugsweise 1 bis 15 Gew.-% - bezogen auf den Feststoffgehalt der Konzentrate - enthalten. Weiterhin können die Mittel, bei denen es sich beispielsweise um Haarshampoos, Haarlotionen, Schaumbäder, Cremes oder Lotionen handelt, ferner als weitere Hilfs- und Zusatzstoffe Co-Tenside, Ölkörper, Co-Emulgatoren, Überfettungsmittel, Stabilisatoren, Wachse, Konsistenzgeber, Verdickungsmittel, Kationpolymere, Siliconverbindungen, biogene Wirkstoffe, Antischuppenmittel, Filmbildner, Konservierungsmittel, Hydrotrope, Solubilisatoren, UV-Adsorber, Farb- und Duftstoffe enthalten.

Typische Beispiele für **Co-Tenside** sind Monoglyceridsulfate, Mono- und/oder Dialkylsulfosuccinate, Fettsäureisethionate, Fettsäuresarcosinate, Fettsäuretauride, Ethercarbonsäuren, Fettsäureglucamide, und/oder Proteinfettsäurekondensate.

Als **Ölkörper** kommen beispielsweise Guerbetalkohole auf Basis von Fettalkoholen mit 6 bis 18, vorzugsweise 8 bis 10 Kohlenstoffatomen, Ester von linearen C₆-C₂₀-Fettsäuren mit linearen C₆-C₂₀-Fettalkoholen, Ester von verzweigten C₆-C₁₃-Carbonsäuren mit linearen C₆-C₂₀-Fettalkoholen, Ester von linearen C₆-C₁₈-Fettsäuren mit verzweigten Alkoholen, insbesondere 2-Ethylhexanol, Ester von linearen und/oder verzweigten Fettsäuren mit mehrwertigen Alkoholen (wie z.B. Dimerdiol oder Trimertriol) und/oder Guerbetalkoholen, Triglyceride auf Basis C₆-C₁₀-Fettsäuren, Ester von C₆-C₂₂-Fettalkoholen und/oder Guerbetalkoholen mit aromatischen Carbonsäuren, insbesondere Benzoesäure, pflanzliche Öle, verzweigte primäre Alkohole, substituierte Cyclohexane, Guerbetcarbonate, Dialkylether und/oder aliphatische bzw. naphthenische Kohlenwasserstoffe sowie Siliconöle in Betracht.

Als Co-Emulgatoren kommen beispielsweise nichtionogene Tenside aus mindestens einer der folgenden Gruppen in Frage:
(b1) Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
(b2) C_{12/18}-Fettsäuremono- und -diester von Anlagerungsprodukten von 1 bis 30 Mol Ethylenoxid an Glycerin;
(b3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
(b4) Anlagerungsprodukte von 15 bis 60 Mol Ethylenoxid an Ricinusöl und/oder gehärtetes Ricinusöl;
(b5) Polyol- und insbesondere Polyglycerinester wie z.B. Polyglycerinpolyricinoleat oder Polyglycerinpoly-12-hydroxystearat. Ebenfalls geeignet sind Gemische von Verbindungen aus mehreren dieser Substanzklassen;
(b6) Anlagerungsprodukte von 2 bis 15 Mol Ethylenoxid an Ricinusöl und/oder gehärtetes Ricinusöl;
(b7) Partialester auf Basis linearer, verzweigter, ungesättigter bzw. gesättigter C_{12/22}-Fettsäuren, Ricinolsäure sowie 12-Hydroxystearinsäure und Glycerin, Polyglycerin, Pentaerythrit, Dipentaerythrit, Zuckeralkohole (z.B. Sorbit) sowie Polyglucoside (z.B. Cellulose);
(b8) Trialkylphosphate;
(b9) Wollwachsalkohole;
(b10) Polysiloxan-Polyalkyl-Polyether-Copolymere bzw. entsprechende Derivate;
(b11) Mischester aus Pentaerythrit, Fettsäuren, Citronensäure und Fettalkohol gemäß **DE-PS 11 65 574** und/oder Mischester von Fettsäuren mit 6 bis 22 Kohlenstoffatomen, Methylglucose und Polyolen, vorzugsweise Glycerin sowie
(b12) Polyalkylenglycole.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE-PS 20 24 051** als Rückfettungsmittel für kosmetische Zubereitungen bekannt.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen. Als **Konsistenzgeber** kommen in erster Linie Fettalkohole mit 12 bis 22 und vorzugsweise 16 bis 18 Kohlenstoffatomen und daneben Partialglyceride in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete **Verdickungsmittel** sind beispielsweise Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono und -diester von Fettsäuren, Polyacrylate, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Geeignete **kationische Polymere** sind beispielsweise kationische Cellulosederivate, kationische Stärke, Copolymere von Diallylammoniumsalzen und Acrylamiden, quaternierte Vinylpyrrolidon/Vinylimidazol-Polymere wie z.B. Luviquat® (BASF AG, Ludwigshafen/ FRG), Kondensationsprodukte von Polyglycolen und Aminen (Polymer J 400, Union Carbide), quaternierte Kollagenpolypeptide wie beispielsweise Lauryldimonium hydroxypropyl hydrolyzed collagen (Lamequat®L, Grünau GmbH), quaternierte Weizenpolypeptide, Polyethylenimin, kationische Siliconpolymere wie z.B. Amidomethicone oder Dow Corning, Dow Corning Co./US, Copolymere der Adipinsäure und Dimethylaminohydroxypropyldiethylentrimamin (Cartaretine®, Sandoz/CH), Polyaminopolyamide wie z.B. beschrieben in der **FR-A 22 52 840** sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen wie z.B. Dibrombutan mit Bisdialkylaminen wie z.B. Bis-Dimethylamino-1,3-propan, kationischer Guar-Gum wie z.B. Jaguar® CBS, Jaguar® C-17, Jaguar® C-16 der Celanese/US, quaternierte Ammoniumsalz-Polymere wie z.B. Mirapol® A-15, Mirapol® AD-1, Mirapol® AZ-1 der Miranol/US.

Geeignete **Siliconverbindungen** sind beispielsweise Dimethylpolysiloxane, Methyl-phenylpolysiloxane, cyclische Silicone sowie amino-, fettsäure-, alkohol-, polyether-, epoxy-, fluor- und/oder alkylmodifizierte Siliconverbindungen, die bei Raumtemperatur sowohl flüssig als auch harzförmig vorliegen können. Typische Beispiele für **Fette** sind Glyceride, als **Wachse** kommen u.a. Bienenwachs, Paraffinwachs oder Mikrowachse gegebenenfalls in Kombination mit hydrophilen Wachsen, z.B. Cetylstearylalkohol oder Partialglyceriden in Frage. Als **Perlglanzwachse** können insbesondere Mono- und Difettsäureester von Polyalkylenglycolen, Partialglyceride oder Ester von Fettalkoholen mit mehrwertigen Carbonsäuren bzw. Hydroxycarbonsäuren verwendet werden. Als **Stabilisatoren** können Metallsalze von Fettsäuren wie z.B. Magnesium-, Aluminium- und/oder Zinkstearat eingesetzt werden. Unter **biogenen Wirkstoffen** sind beispielsweise Bisabolol, Allantoin, Phytantriol, Panthenol, AHA-Säuren, Pflanzenextrakte und Vitaminkomplexe zu verstehen. Als **Antischuppenmittel** können Climbazol, Octopirox und Zinkpyrethion eingesetzt werden. Gebräuchliche **Filmbildner** sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen. Zur Verbesserung des Fließverhaltens können ferner Hydrotrope wie beispielsweise Ethanol, Isopropylalkohol, Propylenglycol oder Glucose eingesetzt werden. Als **Konservierungsmittel** eignen sich beispielsweise Phenoxyethanol, Formaldehydlösung, Parabene, Pentandiol oder Sorbinsäure. Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die fertigen Zubereitungen - betragen. Die Herstellung der Mittel kann durch übliche Kalt- oder Heißprozesse erfolgen; vorzugsweise arbeitet man nach der Phaseninversionstemperatur-Methode.

### Beispiele

Zur Herstellung der Rezepturen R1 bis R6 wurden die wäßrigen tensidischen Emulgatoren vorgelegt, auf 65°C erwärmt und die Perlglanzwachse eingerührt. Die Fließfähigkeit wurde anschließend bei 20°C beurteilt. Dabei bedeutet (+) fließfähig und (-) nicht fließfähig. Die Ergebnisse sind in Tabelle 1 zusammengefaßt. Die Rezepturen R1 bis R4 sind erfindungsgemäß, die Rezepturen R5 und R6 dienen zum Vergleich.

**Tabelle 1**

| **Perlglanzkonzentrate (Mengenangaben als Gew.-%)** | | | | | | |
|---|---|---|---|---|---|---|
| **INCI Bezeichnung** | **R1** | **R2** | **R3** | **R4** | **R5** | **R6** |
| Sodium Laureth-2 Sulfate | 56 | 50 | 56 | 56 | - | - |
| Sodium Laurylsulfate | - | - | - | - | 56 | 56 |
| Cocoamidopropyl Betaine | 20 | 25 | 15 | - | 20 | 20 |
| Lauryl Polyglucose | - | - | - | 15 | - | - |
| Triethylenglycol Distearate | 2 | - | 2 | 2 | 5 | 4 |
| Monoethylenglycol Distearate | - | 4 | - | - | - | - |
| Cocamide DEA | - | - | 2 | 2 | - | - |
| Kochsalz | 4 | 4 | 4 | 4 | 4 | - |
| Glycerin | - | - | - | - | - | 15 |
| Wasser | ad 100 | | | | | |
| ***Stabilität*** | + | + | + | + | - | + |

## Patentansprüche

1. Wäßrige Perlglanzkonzentrate, frei von Polyolen, bestehend aus
(a) 30 bis 80 Gew.-% Alkylethersulfaten,
(b1) 5 bis 20 Gew.-% Betainen und/oder
(b2) 5 bis 20 Gew.-% Alkyl- und/oder Alkenyloligoglykosiden und
(c) 1 bis 10 Gew.-% (Oligo)Ethylenglycolmono- und/oder -difettsäureestem,
mit der Maßgabe, daß sich die Mengenangaben mit Wasser und gegebenenfalls nur noch Elektrolytsalzen zu 100 Gew.-% ergänzen.

2. Konzentrate nach Anspruch 1, **dadurch gekennzeichnet, daß** sie Alkylethersulfate der Formel **(I)** enthalten,
**R**^{**1**}**O-(CH**_{**2**}**CH**_{**2**}**O)**_{**m**}**SO**_{**3**}**X (I)**
in der R¹ für einen linearen oder verzweigten Alkyl- und/oder Alkenylrest mit 6 bis 22 Kohlenstoffatomen, m für Zahlen von 1 bis 10 und X für ein Alkali- und/oder Erdalkalimetall, Ammonium, Alkylammonium, Alkanolammonium oder Glucammonium steht.

3. Konzentrate nach den Ansprüchen 1 und/oder 2, **dadurch gekennzeichnet, daß** sie Betaine der Formel **(II)** enthalten, 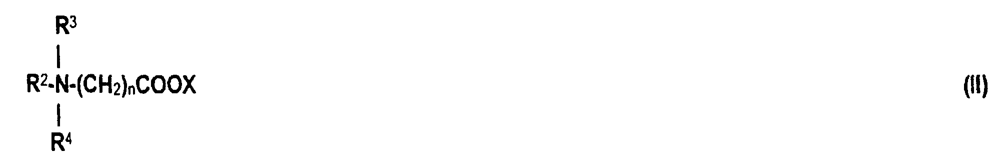 in der R² für Alkyl- und/oder Alkenylreste mit 6 bis 22 Kohlenstoffatomen, R³ für Wasserstoff oder Alkylreste mit 1 bis 4 Kohlenstoffatomen, R⁴ für Alkylreste mit 1 bis 4 Kohlenstoffatomen, n für Zahlen von 1 bis 6 und X für ein Alkali- und/oder Erdalkalimetall oder Ammonium steht.

4. Konzentrate nach mindestens einem der Ansprüche 1 bis 3, **dadurch gekennzeichnet, daß** sie Betaine der Formel **(III)** enthalten, 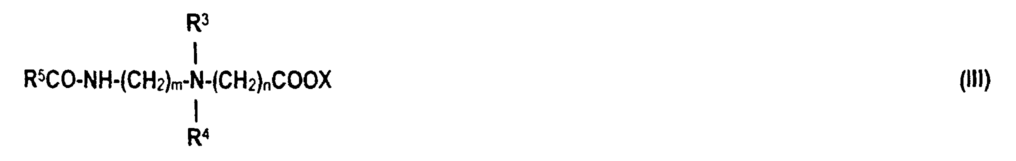 in der R⁵CO für einen aliphatischen Acylrest mit 6 bis 22 Kohlenstoffatomen und 0 oder 1 bis 3 Doppelbindungen, m für Zahlen von 1 bis 3 steht und R³, R⁴, n und X die oben angegebenen Bedeutungen haben.

5. Konzentrate nach mindestens einem der Ansprüche 1 bis 4, **dadurch gekennzeichnet, daß** sie Alkylund/oder Alkenyloligoglykoside der Formel **(IV)** enthalten
**R**^{**6**}**O-[G]**_{**p**} **(IV)**
in der R⁶ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht.

6. Verwendung von Perlglanzkonzentraten nach den Ansprüchen 1 bis 5 zur Herstellung von perlglänzenden oberflächenaktiven Zubereitungen.

## Claims

1. Aqueous polyol-free pearlescing concentrates containing
(a) 30 to 80% by weight alkyl ether sulfates,
(b1) 5 to 20% by weight betaines and/or
(b2) 5 to 20% by weight alkyl and/or alkenyl oligoglycosides and
(c) 1 to 10% by weight (oligo)ethylene glycol mono- and/or difatty acid esters,
with the proviso that the quantities shown add up to 100% by weight, with water and optionally electrolyte salts.

2. Concentrates as claimed in claim 1, **characterized in that** they contain alkyl ether sulfates corresponding to formula (I):
**R**^{**1**}**O-(CH**_{**2**}**CH**_{**2**}**O)**_{**m**}**SO**_{**3**}**X (I)**
in which R¹ is a linear or branched alkyl and/or alkenyl group containing 6 to 22 carbon atoms, m is a number of 1 to 10 and X is an alkali metal and/or alkaline earth metal, ammonium, alkylammonium, alkanolammonium or glucammonium.

3. Concentrates as claimed in claims 1 and 2, **characterized in that** they contain betaines corresponding to formula **(II):** 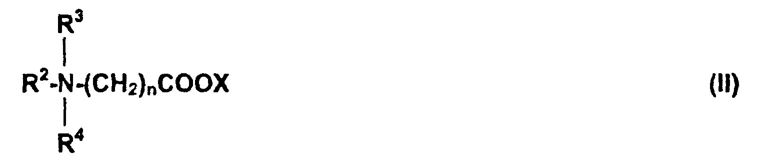 in which R² stands for alkyl and/or alkenyl groups containing 6 to 22 carbon atoms, R³ stands for hydrogen or alkyl groups containing 1 to 4 carbon atoms, R⁴ stands for alkyl groups containing 1 to 4 carbon atoms, n is a number of 1 to 6 and X is an alkali metal and/or alkaline earth metal or ammonium.

4. Concentrates as claimed in claims 1 to 3, **characterized in that** they contain betaines corresponding to formula **(III):** 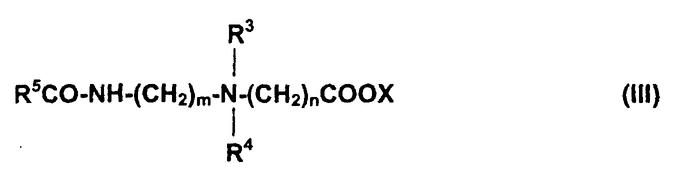 in which R⁵CO is an aliphatic acyl group containing 6 to 22 carbon atoms and 0 or 1 to 3 double bonds, m is a number of 1 to 3 and R³, R⁴, n and X are as defined above.

5. Concentrates as claimed in claims 1 to 4, **characterized in that** they contain alkyl and/or alkenyl oligoglycosides corresponding to formula **(IV):**
**R**^{**6**}**O-[G]**_{**p**} **(IV)**
where R⁶ is an alkyl and/or alkenyl group containing 4 to 22 carbon atoms, G is a sugar unit containing 5 or 6 carbon atoms and p is a number of 1 to 10.

6. The use of the pearlescing concentrates claimed in claims 1 to 5 for the production of pearlescent surface-active compositions.

## Revendications

1. Concentrés aqueux de lustre nacré, dépourvus de polyols, constitués de
(a) 30 à 80 % en poids de sulfates d'alkyléthers,
(b1) 5 à 20 % en poids de bêtaïnes et/ou
(b2) 5 à 20 % en poids d'alkyl- et/ou alcényloligoglycosides et
(c) 1 à 10 % en poids de mono- et/ou diesters d'acides gras et d'(oligo)éthylèneglycol,
étant précisé que les indications quantitatives se complètent à 100 % en poids avec de l'eau et éventuellement seulement des sels électrolytiques.

2. Concentrés selon la revendication 1,
**caractérisés en ce qu'**
ils contiennent des sulfates d'alkyléthers de formule (I)
R¹O-(CH₂CH₂O)ₘSO₃X (I)
dans laquelle R¹ représente un radical alkyle et/ou alcényle linéaire ou ramifié comportant de 6 à 22 atomes de carbone, m représente des nombres allant de 1 à 10 et X représente un métal alcalin et/ou alcalino-terreux, l'ammonium, un alkylammonium, un alcanolammonium ou un glucammonium.

3. Concentrés selon les revendications 1 et/ou 2,
**caractérisés en ce qu'**
ils contiennent des bêtaïnes de formule (II) 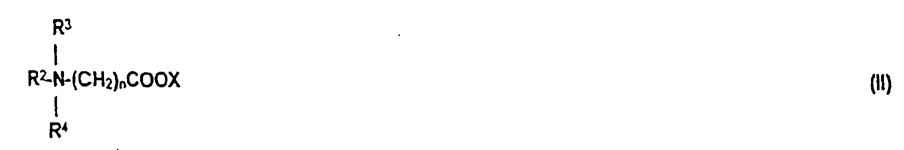 dans laquelle R² représente des radicaux alkyle et/ou alcényle comportant de 6 à 22 atomes de carbone, R³ représente un hydrogène ou des radicaux alkyle comportant de 1à 4 atomes de carbone, R⁴ représente des radicaux alkyle comportant de 1 à 4 atomes de carbone, n représente des nombres allant de 1 à 6 et X représente un métal alcalin et/ou alcalino-terreux ou l'ammonium.

4. Concentrés selon au moins une des revendications 1 à 3,
**caractérisés en ce qu'**
ils contiennent des bêtaïnes de formule (III) 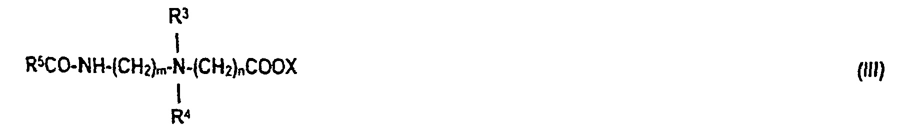 dans laquelle R⁵CO représente un radical acyle aliphatique comportant de 6 à 22 atomes de carbone et 0 ou 1 à 3 doubles liaisons, m représente des nombres allant de 1 à 3 et R³, R⁴, n et X ont les significations indiquées ci-dessus.

5. Concentrés selon au moins une des revendications 1 à 4,
**caractérisés en ce qu'**
ils contiennent des alkyl- et/ou alcényloligoglycosides de formule (IV)
R⁶O-[G]ₚ (IV)
dans laquelle R⁶ représente un radical alkyle et/ou alcényle comportant de 4 à 22 atomes de carbone, G représente un radical saccharique comportant 5 ou 6 atomes de carbone et p représente des nombres allant de 1à 10.

6. Utilisation de concentrés de lustre nacré selon les revendications 1 à 5 pour la fabrication de préparations tensioactives à lustre nacré.
